(19) Europäisches Patentamt
European Patent Office
Office européen des brevets

(11) Publication number: **0 260 655 A2**

# (12) EUROPEAN PATENT APPLICATION

(21) Application number: 87113474.8

(22) Date of filing: 15.09.87

(51) Int. Cl.[4]: C07C 103/58 , C07D 295/18 , C07C 69/732 , C07C 69/738 , A61K 31/215 , A61K 31/16

(30) Priority: 16.09.86 JP 218547/86

(43) Date of publication of application: 23.03.88 Bulletin 88/12

(84) Designated Contracting States: AT BE CH DE ES FR GB GR IT LI LU NL SE

(71) Applicant: SUMITOMO PHARMACEUTICALS COMPANY, LIMITED
40, Dosho-machi 2-chome
Higashi-ku Osaka-shi Osaka-fu(JP)

(72) Inventor: Nakamura, Toshio
11-16, Koshiengobancho
Nishinomiya-shi(JP)
Inventor: Kawakami, Hajime
3-32-702, Fukazucho
Nishinomiya-shi(JP)
Inventor: Ono, Keiichi
10-4, Momoyamadai-3-cho
Sakai-shi(JP)

(74) Representative: Vossius & Partner
Siebertstrasse 4 P.O. Box 86 07 67
D-8000 München 86(DE)

## (54) Unsaturated fatty acid derivatives and their production.

(57) A compound of the formula:

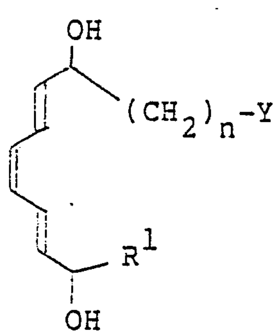

wherein Y is a free or esterified carboxyl group, or a group of the formula:

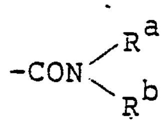

EP 0 260 655 A2

(wherein $R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, an $C_1$-$C_{12}$ alkyl group substituted with a group of the formula:

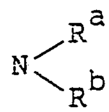

(wherein $R^c$ and $R^d$ are each independently a hydrogen atom, or a $C_1$-$C_4$ alkyl group), a $C_3$-$C_{10}$ heterocyclic group, a phenyl group optionally substituted with one to three substituents selected from the group consisting of halogen atom, hydroxyl group, $C_1$-$C_4$ alkyl group, trifluoromethyl group, $C_1$-$C_4$ alkoxy group, and group of the formula:

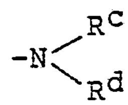

wherein $R^c$ and $R^d$ are as defined above) or a group of the formula:

A-B

[wherein A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_1$-$C_{12}$ alkylthio group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with one to three substituents selected from the group consisting of halogen atom, hydroxy group, $C_1$-$C_4$ alkyl group, group of the formula:

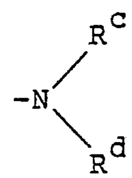

(wherein $R^c$ and $R^d$ are as defined above), trifluoromethyl group, $C_1$-$C_4$ alkylthio group and $C_1$-$C_4$ alkoxy group] or a pharmaceutically acceptable salt thereof. Said compound has potent anti-leucotriene $B_4$ action, and is useful in the treatment of inflammatory states or immunological disorders.

## UNSATURATED FATTY ACID DERIVATIVES AND THEIR PRODUCTION

The present invention relates to novel unsaturated fatty acids and to their production.

More particularly, this invention relates to novel unsaturated fatty acids, to processes for producing those fatty acids and to pharmaceutical compositions containing at least one of those unsaturated fatty acids, which have excellent anti-leucotriene $B_4$ activity and are useful as an anti-allergic agent and an anti-inflammatory agent.

In 1979 B. Sammuelsson reported the isolation and biological effects of leucotrienes (B. Sammuelsson et al. (1980): In: Advances in Prostaglandin and Thromboxane Research, Vol. 6, edited by B. Sammuelsson, R. Ramwell, and R. Paaletti, P. I. Raven Press, New York).

Since then, a tremendous amount of research in the synthetic organic chemistry and pharmacology of leucotriene $A_4$, $B_4$, $C_4$, $D_4$, etc. has been performed.

Leucotrienes induce an increase in capillary permeability and cause smooth muscle contraction. Leucotriene $B_4$, one of leucotrienes which is shown below, has different pharmacological properties than the others. It is chemotactic for macrophages and neutrophils at concentrations of $\sim$1 ng/ml (greater than any, other known lipid chemotactic factor). It is detected in the synovia of patients with rheumatoid arthritis or gouty arthritis, and in the sputum of obstructive airways diseases which suggest that it is a primary mediator of inflammatory and allergic states.

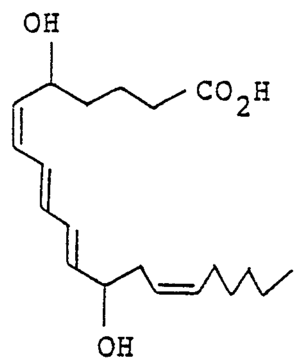

Leucotriene $B_4$

In accordance with the present invention, novel unsaturated fatty acids of the following general formula [I] and their non-toxic pharmaceutically acceptable salts are provided, which have potent anti-leucotriene $B_4$ activity which include suppression of chemotaxis, degranulation and $O_2$-production of leukocytes, and modulation of lymphocytes activity, etc. This action may render these compounds very useful as drugs for the treatment of inflammatory states or immunological disorders such as allergy, rheumatoid arthritis, inflammatory bowel disease and cancer.

The novel unsaturated fatty acids provided by the present invention are those represented by the formula [I]:

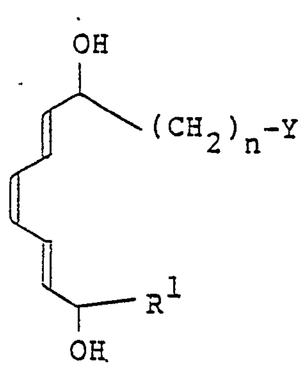

[I]

wherein Y is a free or esterified carboxyl group, or a group of the formula:

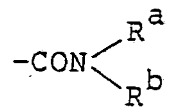

(wherein $R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, a $C_1$-$C_{12}$ alkyl group substituted with a group of the formula:

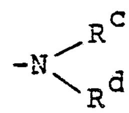

(wherein $R^c$ and $R^d$ are each independently a hydrogen atom, or a $C_1$-$C_4$ alkyl group), a $C_3$-$C_{10}$ heterocyclic group, a phenyl group optionally substituted with one to three substituents selected from the group consisting of halogen atom, hydroxyl group, $C_1$-$C_4$ alkyl group, trifluoromethyl group, $C_1$-$C_4$ alkoxy group, and group of the formula:

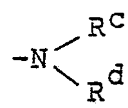

(wherein $R^c$ and $R^d$ are as defined above) or a group of the formula:

A-B

[wherein A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_1$-$C_{12}$ alkylthio group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with one to three substituents selected from the group consisting of halogen atom, hydroxy group, $C_1$-$C_4$ alkyl group, group of the formula:

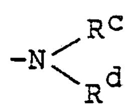

(wherein $R^c$ and $R^d$ are as defined above), trifluoromethyl group, $C_1$-$C_4$ alkylthio group and $C_1$-$C_4$ alkoxy group]; n is 2, 3 or 4.

In the definitions as used above, the term "halogen" includes fluorine, chlorine, bromine and iodine; the terms "$C_1$-$C_4$ alkyl", "$C_1$-$C_4$ alkoxy" and "$C_1$-$C_4$ alkylthio" each means a straight or branched chain alkyl, alkoxy or alkylthio group having from 1 to 4 carbon atoms (e.g. methyl, ethyl, n-propyl, isopropyl, n-butyl, methoxy, ethoxy, n-propoxy, methylthio, ethylthio, isopropoxy, n-butoxy, etc.).

The term "$C_1$-$C_{12}$ alkyl" in the following cases, "$C_1$-$C_{12}$ alkyl" and "$C_1$-$C_{12}$ alkyl in the $C_1$-$C_{12}$ alkoxy group" and "$C_1$-$C_{12}$ alkyl in the $C_1$-$C_{12}$ alkylthio group", means a straight or branched chain alkyl group having from one to 12 carbon atoms (e.g. methyl, ethyl, n-propyl, isopropyl, n-butyl, isobutyl, n-pentyl, isopentyl, 1-methylpentyl, 2-methylpentyl, 1,1-dimethylpentyl, 1-ethylpentyl, 2-ethylpentyl, n-hexyl, 1-methylhexyl, 2-methylhexyl, 3-methylhexyl, heptyl, 1-methylheptyl, 2-methylheptyl, 1-ethylheptyl, 2-ethylheptyl, n-octyl, 1-methyloctyl, 2-methyloctyl, 1-ethyloctyl, 2-ethyloctyl, 2,6-dimethylheptyl, 1,6-dimethylheptyl, n-nonyl, 1-methylnonyl, 2-methylnonyl, n-decyl, 1-methyldecyl, 2-methyldecyl, 2-ethyldecyl etc.).

The terms "$C_2$-$C_{12}$ alkenyl" and "$C_2$-$C_{12}$ alkynyl" each means a straight or branched chain alkenyl or alkynyl group having from 2 to 12 carbon atoms (e.g. vinyl, 2-propenyl, 2-butenyl, 2-pentenyl, 2-hexenyl, 5-heptenyl, 6-methyl-5-heptenyl, 2,6-dimethyl-5-heptenyl, 3-pentenyl, 4-pentenyl, 2,6-dimethyl-5-octenyl, 1,1,6-trimethyl-5-heptenyl, 4,8-dimethyl-7-nonenyl, 2,6-dimethyl-1,5-heptadienyl, 2-propynyl, 1-methylenepentyl, 2-butynyl, 2-pentynyl, 3-pentynyl, 1-methyl-3-pentynyl, 4-pentynyl 4-hexynyl, 5-heptynyl, 6-heptynyl, 2-methyl-5-heptynyl, etc.).

The term "$C_3$-$C_{10}$ cycloalkyl" in the both cases, "$C_3$-$C_{10}$ cycloalkyl" and "$C_3$-$C_{10}$ cycloalkyl in the $C_3$-$C_{10}$ cycloalkoxy group" means a cyclic alkyl group which is optionally substituted with a $C_1$-$C_4$ alkyl or alkenyl group and which has from 3 to 10 carbon atoms (e.g. cyclopropyl, cyclobutyl, cyclopentyl, cyclohexyl, cycloheptyl, 2-isopropylidenemethyl-3,3-dimethylcyclopropyl, 2-propylcyclopropyl, 3-ethylcyclobutyl, 3-ethylcyclopentyl, 4-methylcyclohexyl, 3-ethylcyclohexyl, 4-methylcycloheptyl, 2-isopropyl-5-methylcyclohexyl, norbornyl, adamantyl etc.).

The term "$C_4$-$C_{10}$ cycloalkenyl" in the both cases, $C_4$-$C_{10}$ cycloalkenyl" and "$C_4$-$C_{10}$ cycloalkenyl in the $C_4$-$C_{10}$ cycloalkenyloxy" means a cyclic alkenyl group having from 4 to 10 carbon atoms (e.g. bicyclo[4,3,0]-nona-3-en-8-yl, 3-cyclopentenyl, 3-cyclohexenyl, 3-cycloheptenyl, tetrahydro-2-indanyl etc.).

The term "hydroxy $C_1$-$C_{12}$ alkyl" means a straight or branched alkyl group which has from one to 12 carbon atoms and which is substituted with a hydroxy group (e.g. hydroxymethyl, 2-hydroxyethyl, 3-hydroxypropyl, 4-hydroxybutyl, 5-hydroxypentyl, 6-hydroxyhexyl, 7-hydroxyheptyl, 8-hydroxyoctyl, 10-hydroxydecyl, 5-hydroxyhexyl, 4-hydroxypentyl, 5-hydroxy-1,1-dimethylpentyl, 5-hydroxy-2-methylpentyl, 5-hydroxy-1-methylpentyl, 6-hydroxy-2-methylhexyl etc.).

The term "$C_3$-$C_{10}$ heterocyclic group" means a monocyclic or dicyclic group having from 3 to 10 carbon atoms and at least one of hetero atoms selected from nitrogen atoms, sulfur atoms, and oxygen atoms (e.g. piperidine, morpholine, pyrrolidine, piperazine, tetrahydrofuran, tetrahydrothiophene, furan, thiophene, imidazole, pyridine, oxazole, isooxazole, pyrrole, pyrazole, pyrimidine, indole, benzofuran, purine, benzothiophene, quinoline, pyrrolidone, dihydrothiophene, dihydrobenzofuran, 1,4-benzodioxane, etc.).

The term "$C_1$-$C_7$ alkylene" means a straight or branched alkylene chain having from one to 7 carbon atoms (e.g. methylene, ethylene, trimethylene, tetramethylene, pentamethylene, hexamethylene, heptamethylene, methylmethylene, dimethylmethylene, 1,1-dimethylethylene, 2-methyltetramethylene, 1-methylpentamethylene, 2-methylhexamethylene, 1-ethylethylene, 2-ethylethylene, 2-ethyltrimethylene, etc.).

The term "5 to 7 membered saturated heterocyclic group" includes piperidine, morpholine, pyrrolidine, homopiperidine, piperazine, N-($C_1$-$C_4$) alkylpiperazine, etc.

The term "$C_3$-$C_7$ cycloalkyl" means a cyclic alkyl group which is optionally substituted with a $C_1$-$C_4$ alkyl group and which has from 3 to 7 carbon atoms (e.g. cyclopropyl, cyclobutyl, cyclopentyl, cyclohexyl, cycloheptyl, 3-ethylcyclopentyl, 4-methylcyclohexyl, etc.).

The term "esterified carboxyl group" includes $C_1$-$C_4$ alkoxycarbonyl, aryloxycarbonyl (e.g. phenoxycarbonyl, naphthoxycarbonyl), aralkyloxycarbonyl (e.g. benzyloxycarbonyl, phenethyloxycarbonyl), ($C_1$-$C_4$ alkoxy)methoxycarbonyl, ($C_2$-$C_5$ alkanoyloxy)methoxycarbonyl (e.g. acetoxymethoxycarbonyl), ($C_3$-$C_7$ cycloalkyloxy)carbonyl, arylcarbonylmethoxycarbonyl and (hydroxy $C_1$-$C_4$ alkoxy)carbonyl.

Accordingly, a basic object of the present invention is to provide the novel unsaturated fatty acids [I] having excellent pharmacological activities.

Another object of the present invention is to provide processes for producing those compounds [I]. A further object of the present invention is to provide a pharmaceutical composition containing a compound of the formula [I].

The novel unsaturated fatty acids [I] of the invention can be prepared by the following two methods:

The compound of the formula [I] can be obtained from a carbonyl compound of the formula [II]:

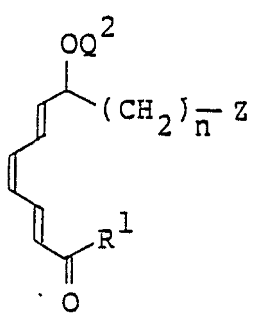

[II]

wherein $Q^2$ is a hydrogen atom or an acyl group, Z is an esterified carboxyl group or a group of the formula:

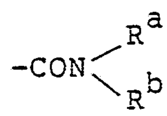

(wherein $R^a$ and $R^b$ are as defined above), and $R^1$ and n are as defined above, by reacting the latter with a reducing agent, optionally followed by deprotection of a protected hydroxyl group, hydrolysis of an ester group, esterification of a carboxyl group, amidation of a free or esterified carboxyl group, and/or transesterification.

In the definitions as used above, the term "an acyl group" includes a $C_1$-$C_4$ alkanoyl group, a benzoyl group and a substituted benzoyl group (e.g. acetyl, propionyl, benzoyl, p-phenyl benzoyl, 2,4,6-trimethyl benzoyl etc.).

In another way, the compound of the formula [I] can be obtained from a carbonyl compound of the formula [III]:

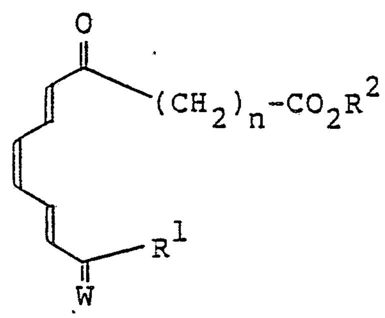

[III]

wherein $R^1$ and n are each as defined above, $R^2$ is a $C_1$-$C_4$ alkyl group and W is an oxygen atom or a group of the formula:

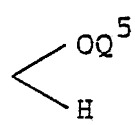

(wherein $Q^5$ is an acyl group or a substituted alkoxymethyl group which forms acetal with the adjoining oxygen atom), by reacting the latter with a reducing agent, optionally followed by deprotection of a protected hydroxyl group, hydrolysis of an ester group, esterification of a carboxyl group, amidation of a free or esterified carboxyl group, and/or transesterification. In the significances as used above, the term "a substituted alkoxymethyl group" means a protective group of a hydroxyl group such as tetrahydropyranyl, tetrahydrofuranyl, 1-ethoxyethyl etc.

Certain of the unsaturated fatty acids [I] can be also prepared by the following method.

Compounds of formula [IV]:

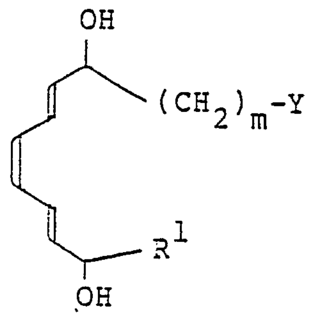

[IV]

wherein $R^1$ and Y are each as defined above, and m is 3 or 4, which are included within the unsaturated fatty acids [I], can be obtained from a carbonyl compound of the formula [V]:

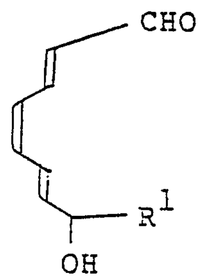

[V]

wherein $R^1$ is as defined above, by reacting the latter with an organometallic compound of the formula [VI]:

$M^1$-$(CH_2)_m$-$C(OR^3)_3$ [VI]

wherein $M^1$ is a lithium atom or a group of the formula: MgX (X is a chlorine atom, a bromine atom or an iodine atom), $R^3$ is a $C_1$-$C_4$ alkyl group, and m is 3 or 4, followed by transformation of an orthoester group to an ester group, and optionally followed by hydrolysis of an ester group, esterification of a carboxyl group, amidation of a free or esterified carboxyl group and/or transesterification.

The sequence of the steps from the carbonyl compounds [II], [III] and [V] to the respective unsaturated fatty acids [I], [I] and [IV] may be represented by the following scheme:

a)

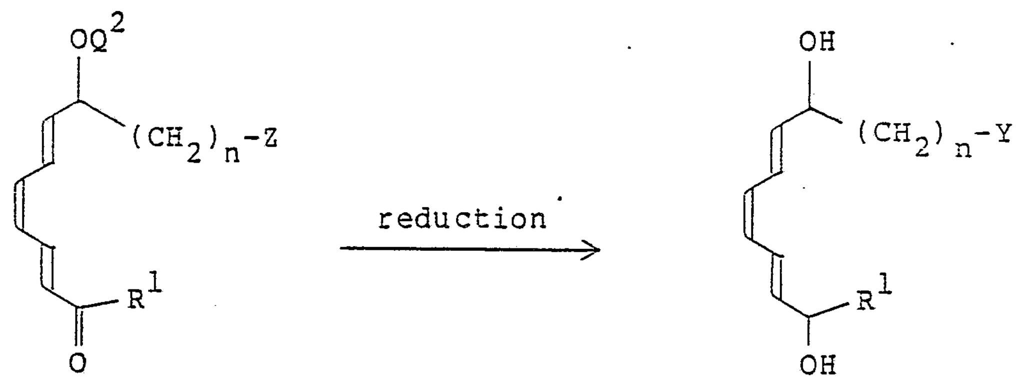


[II] [I]

b)

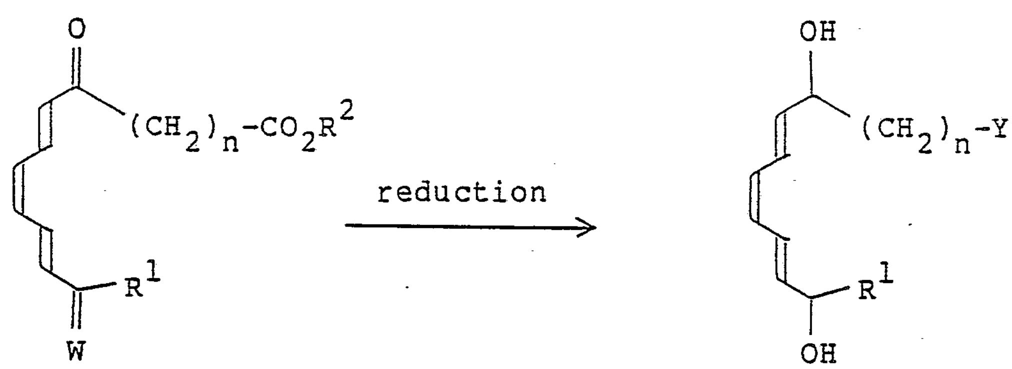


[III] [I]

c)

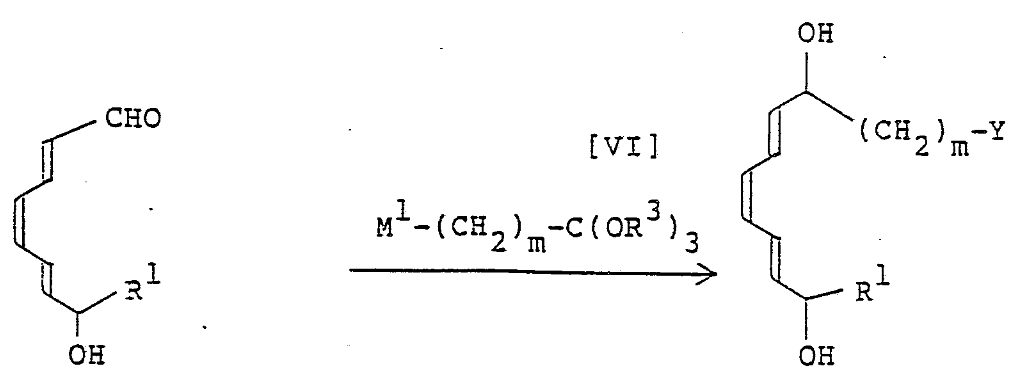


[V] [IV]

Scheme A

STEP 1.

Reduction of the carbonyl compounds [II] and [III] to the respective unsaturated fatty acids [I].

The carbonyl compound [II] or [III] can be converted into the corresponding alcohol by reacting the former with a reducing agent in an inert solvent (e.g. THF, ether, dimethoxyethane, pentane, hexane, benzene, toluene, methanol, ethanol, etc.) at a temperature in the range from -78°C to a room temperature.

As the reducing agent, there may be used for example trialkylborohydride (e.g. lithium triisobutylborohydride), bis(2,4,6-tri-t-butylphenoxy) aluminum hydride, sodium borohydride, zinc borohydride, diisobutyl aluminum hydride, ethoxy 1,1'-binaphthyl-2,2'-dioxyaluminum lithium hydride, sodium trimethoxyaluminum hydride, sodium trimethoxyborohydride, etc.

The deprotection of a protected hydroxyl group can be carried out by the conventional procedure [Protective Group in Organic Chemistry. Edited by J. F. W. McOmie (1973) 95-143].

STEP 2.

Reaction of the aldehyde [V] with an organometallic compound [VI]

The aldehyde [V] can be converted into the corresponding intermediate ortho-ester by reacting of the former with an organometallic compound [VI] in an inert solvent (e.g. ether, THF) at a temperature in the range from -78°C to a room temperature. The organometallic compound [VI] can be prepared by reacting a halide of the formula[VII]:

$X\text{-}(CH_2)_m\text{-}C(OR^3)_3$ [VII]

wherein X, m and $R^3$ are each as defined above, with lithium or magnesium. If magnesium is selected in this reaction, active magnesium is preferably used [J. Org. Chem., 46 4323 (1981)]. The halide [VII] can be prepared by the method of the Literature [R. H. DeWolfe, Synthesis, 1974, 153].

The transformation of an orthoester group to an ester group can be carried out by treating an orthoester compound with an acid (e.g. hydrogen chloride, silica gel, etc.) in an inert solvent (e.g. methanol, THF, ethyl acetate, etc.) at a temperature in the range from -30°C to a room temperature.

The steps of hydrolysis of an esterified carboxyl group, esterification of a carboxyl group, amidation of a free or esterified carboxyl group and transesterification may be represented by the following scheme:

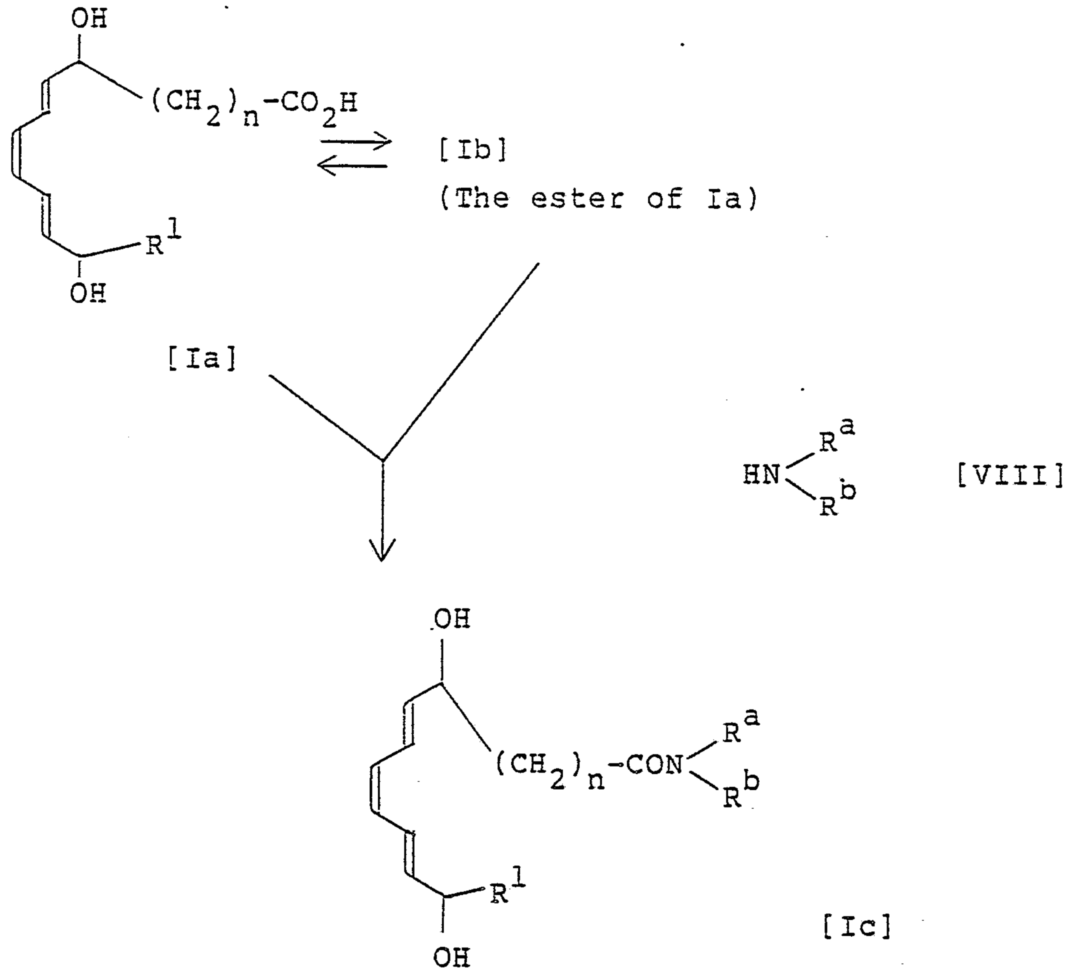

[Ib] (The ester of Ia)

[Ia]

[VIII]

[Ic]

Scheme B

Amidation of an esterified carboxyl group can be carried out by treating an ester compound [Ib] with an amine [VIII] in an inert solvent (e.g. DMF, methanol, ethanol, THF, water) at a temperature in the range from a room temperature to the boiling temperature of the solvent.

Amidation of a carboxyl compound [Ia], hydrolysis of an ester compound [Ib], esterification of a carboxyl compound [Ia], and transesterification of an ester compound [Ib] can be carried out by conventional procedures.

The carbonyl compound [II] used as an intermediate in the present invention can be prepared by the two methods shown in the following scheme:

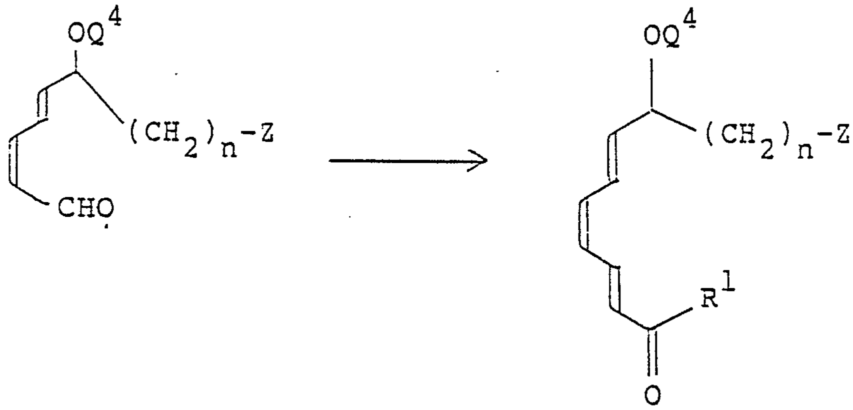

[IX] [IIa]

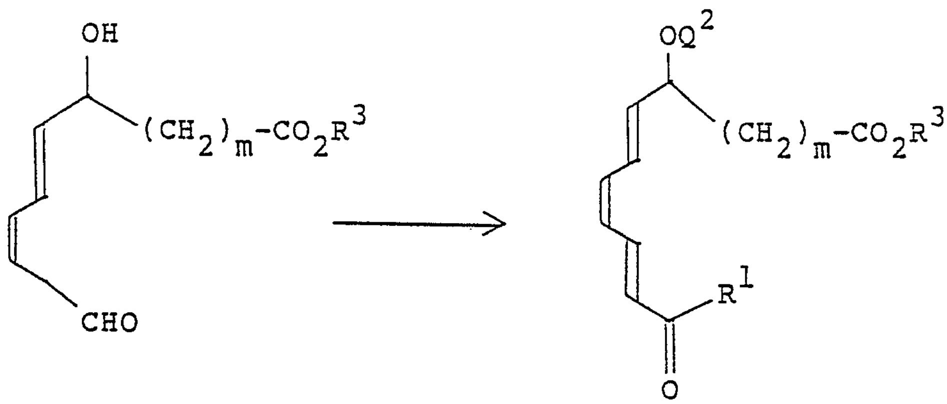

[X] [IIb]

Scheme C

In the formulas illustrated in the scheme C, $R^1$, $R^3$, n, m, $Q^2$ and Z are each as defined above, and $Q^4$ is an acyl group.

Witting reaction of the aldehyde [IX] into the carbonyl compound [IIa] which is included within [II], can be accomplished by reacting the former with a compound of the formula [XI] or [XII]:

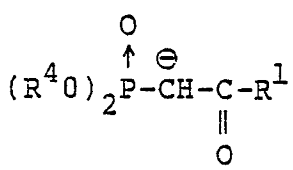

[XI]

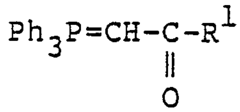

[XII]

wherein $R^4$ is a $C_1$-$C_4$ alkyl group, Ph is a phenyl group, and $R^1$ is as defined above, in an inert solvent (e.g. dioxane, ether, THF, dimethoxyethane, benzene, toluene, n-hexane, DMSO) at a temperature in the range from -30°C to the boiling temperature of the solvent, optionally followed by deprotection of a protected hydroxyl group.

Wittig Reaction of the aldehyde [X] into the carbonyl compound [IIb] which included within [II], can be carried out by the same method as used in the synthesis of the carbonyl compound [IIa] from the aldehyde [IX], optionally followed by protection of a hydroxyl group.

The aldehyde [IX] can be prepared from a diol compound [XIII] by the process shown in the following shceme:

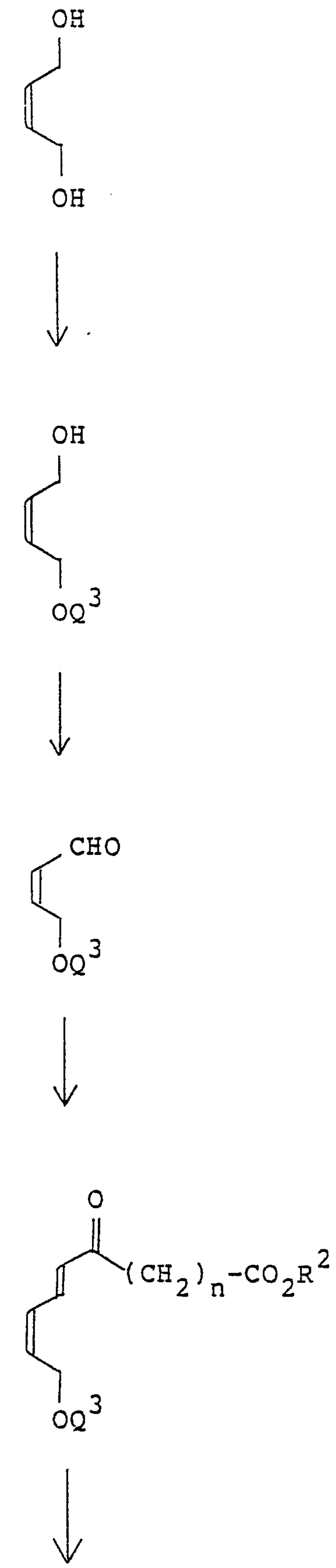

[XIII]

[XIV]

[XV]

[XVI]

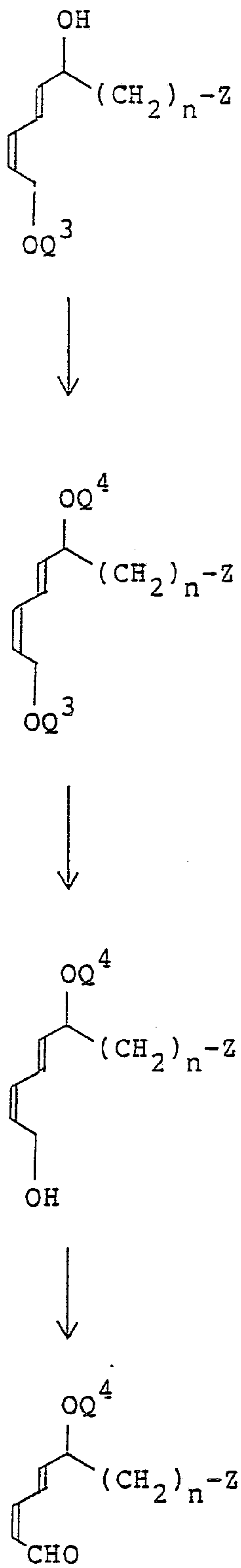

[XVII]

[XVIII]

[XIX]

[IX]

Scheme D

In the formulas illustrated in the scheme D, $Q^3$ is a substituted alkoxymethyl group which forms acetal with the adjoining oxygen atom, and $R^2$, n, z and $Q^4$ are each as defined above.

Detailed explanation of the scheme D is as follows:

Mono protection of the diol [XIII] into the compound [XIV] can be accomplished by treating the former with a reagent which makes an acetal by reaction with a hydroxy group (e.g. 3,4-dihydro-2H-pyran, 5,6-dihydro-4-methoxy-2H-pyran, ethyl vinyl ether, etc.) in the presence of the catalytic amount of an acid (e.g. sulfuric acid, p-toluenesulfonic acid, pyridinium 4-toluenesulfonate) in an inert solvent (e.g. methylene chloride, THF) at a temperature in the range from 0°C to 50°C.

Oxidation of the compound [XIV] into the aldehyde [XV] can be carried out by reacting the former with active manganese dioxide in an inert solvent (e.g. methylene chloride, chloroform, benzene) at a temperature in the range from 0 to 30°C.

The ester [XVI] can be obtained from the aldehyde [XV] by reacting the latter with the compound of the formula [XX] or [XXI]:

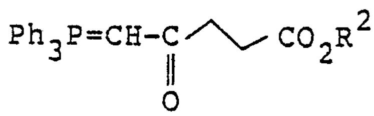
[XX]

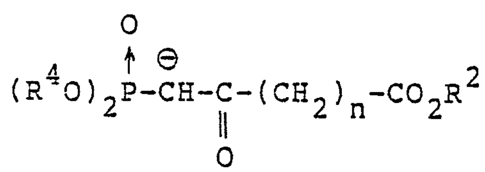
[XXI]

wherein Ph, $R^2$, $R^4$ and n are each as defined above, in an inert solvent (e.g. N,N-dimethylformamide, THF, ether, dimethoxyethane, benzene, toluene, n-hexane, DMSO) at a temperature in the range from -30°C to the boiling temperature of the solvent.

The ester [XVI] can be converted into the compound [XVII] by the same procedure as used in the synthesis of the unsaturated fatty acids [I] from the carbonyl compound [II] or [III].

The compound [XVII] can be converted into the compound [XVIII] by treating the former with an acyl halide or an acid anhydride (e.g. acetic anhydride, acetyl chloride, benzoyl chloride, propionyl chloride) in the presence of a base (e.g. pyridine, triethylamine, 4-(N,N-dimethyl)-aminopyridine) in an inert solvent (e.g. methylene chloride, THF) at a temperature in the range from 0°C to 50°C.

The compound [XVIII] can be converted into the compound [XIX] by conventional procedures.

The compound [XIX] can be converted into the aldehyde [IX] by the same procedure as used in the synthesis of the aldehyde [XV] from the compound [XIV].

The aldehyde [X] can be prepared from the aldehyde [XXII] by the process shown in the following - scheme:

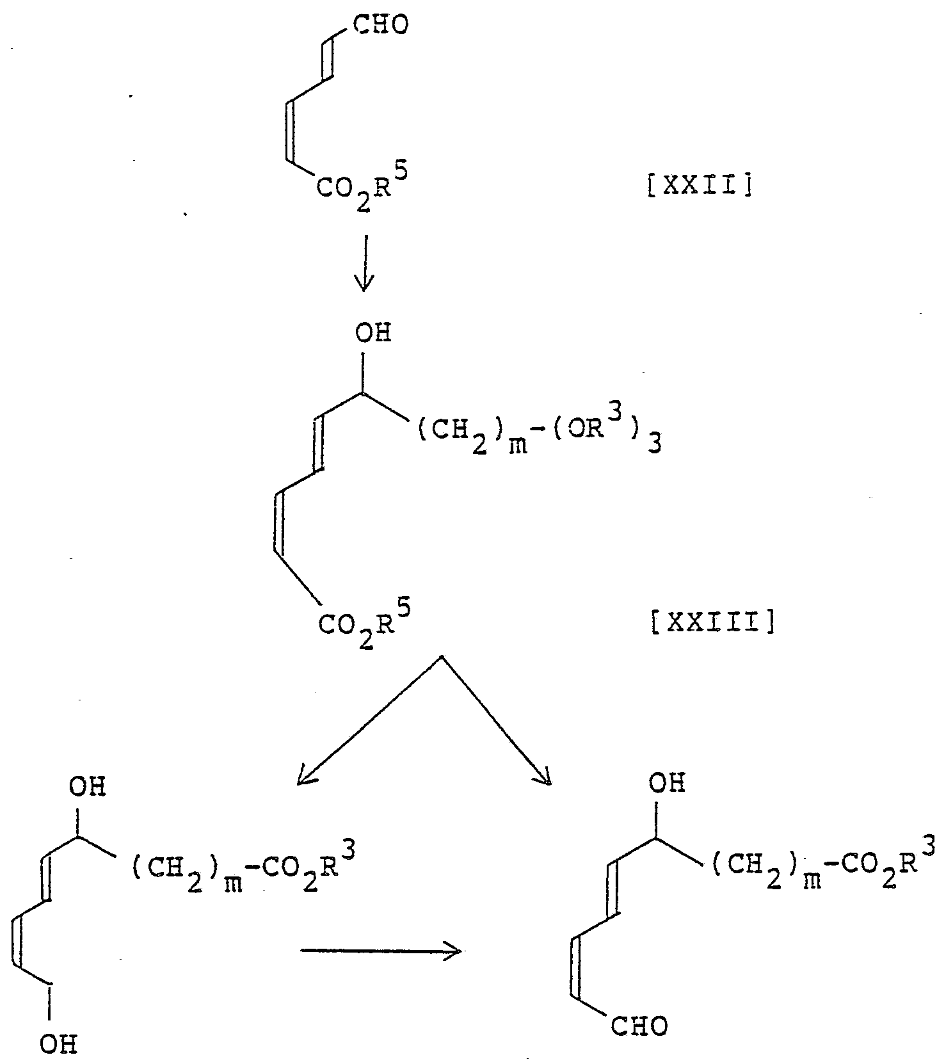

[XXII]

[XXIII]

[XXIV] [X]

Scheme E

In the formulas illustrated in the scheme E, $R^5$ is a $C_1$-$C_4$ alkyl group, and m and $R^3$ are each as defined above.

Detailed explanation of the scheme E is as follows:

The ester [XXIII] can be obtained from the aldehyde [XXII] by the same procedure as used in the synthesis of the unsaturated fatty acids [IV] from the aldehyde [V].

Partial reduction of the ester [XXIII] into the aldehyde [X] can be carried out by reacting the former with a reducing agent (e.g. diisobutylaluminum hydride) in an inert solvent (e.g. n-hexane, toluene, tetrahydrofuran) at a temperature in the range from -78°C to -30°C, followed by treating the resulting compound with an acid (e.g. hydrogen chloride, silica gel) in an inert solvent (e.g. methanol, THF, ethyl acetate) at a temperature in the range from -30°C to room temperature.

The ester [XXIII] can be converted into the alcohol [XXIV] by reacting the former with a reducing agent (e.g. sodium trimethoxyaluminum hydride, lithium tri-s-butylborohydride, diisobutylaluminum hydride) in an inert solvent (e.g. THF, ether) at a temperature in the range from -78°C to room temperature, followed by treating the resulting compound with an acid (e.g. hydrogen chloride, silica gel) in an inert solvent (e.g. methanol, THF, ethyl acetate) at a temperature in the range from -30°C to room temperature.

The alcohol [XXIV] can be converted into the aldehyde [X] by reacting the former with active manganese dioxide in an inert solvent (e.g. methylene chloride, chloroform, benzene, ethyl acetate) at a temperature in the range from 0°C to 30°C.

The carbonyl compound [III] used as an intermediate in the present invention can be prepared by the following scheme:

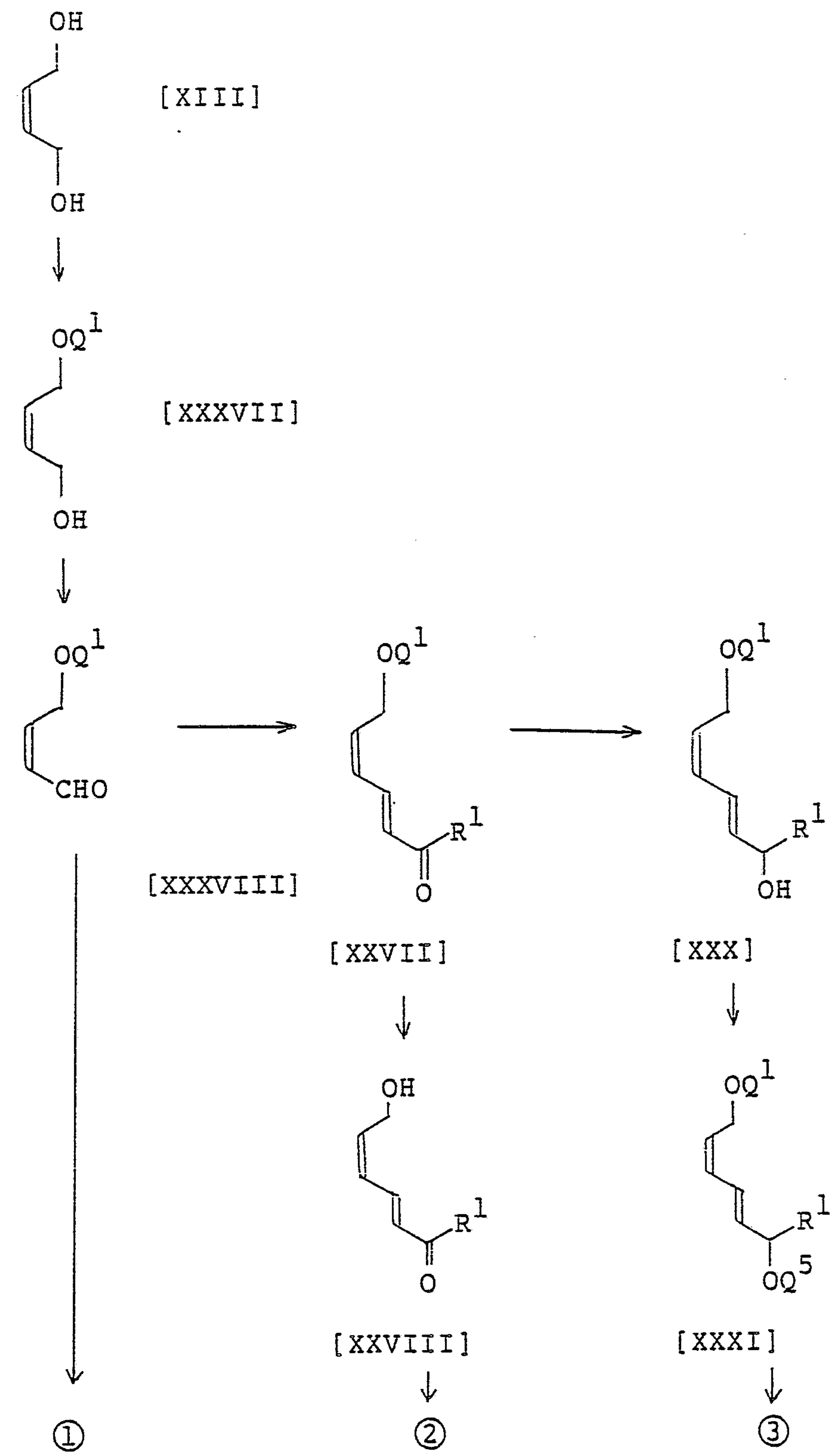

[XIII]

[XXXVII]

[XXXVIII]

[XXVII]

[XXX]

[XXVIII]

[XXXI]

① ② ③

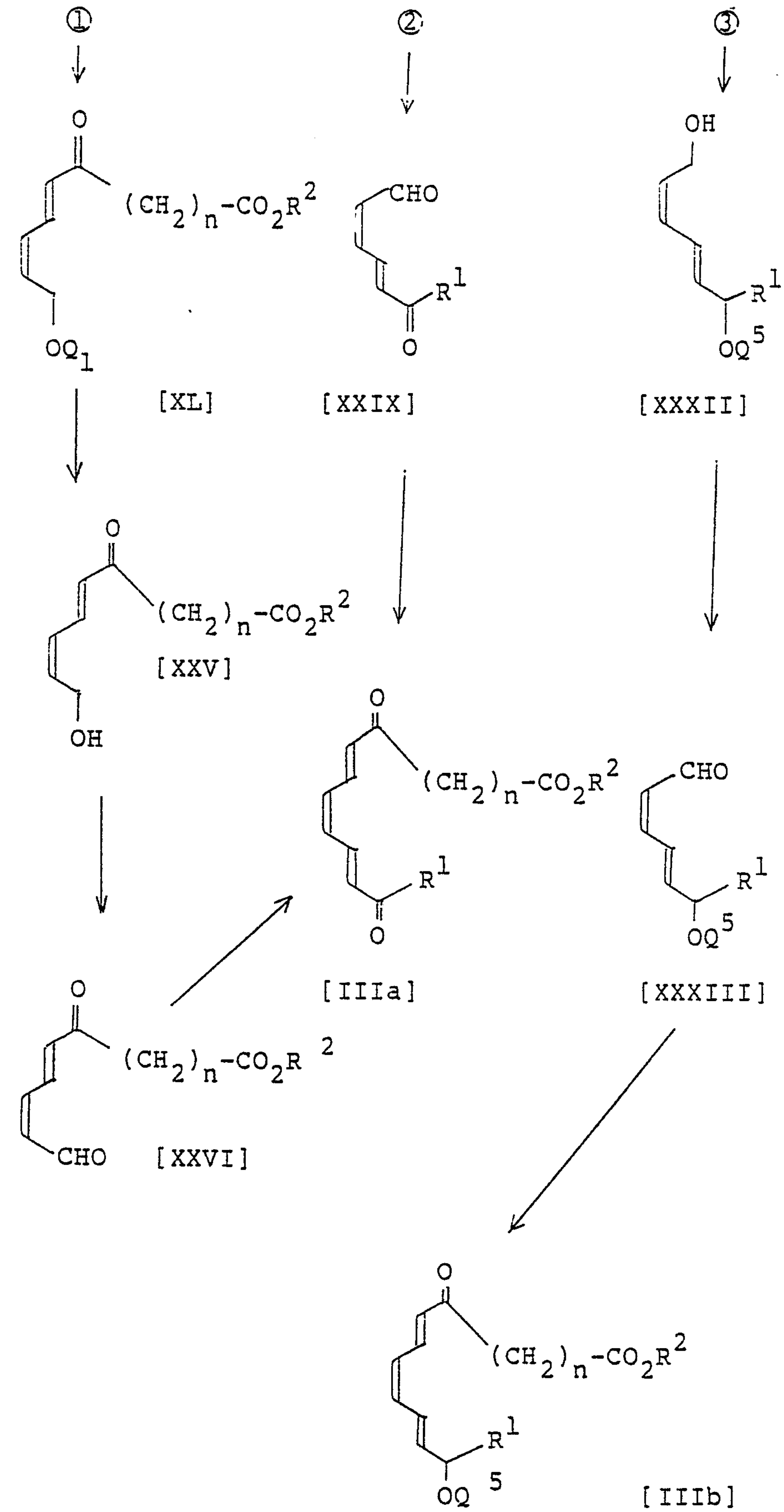

[XL] [XXIX] [XXXII]

[XXV]

[IIIa] [XXXIII]

[XXVI]

[IIIb]

Scheme F

In the formulas illustrated in the scheme F, $R^1$, $R^2$, and n are each as defined above, and $Q^1$ is an acyl group, when $Q^5$ is a substituted alkoxymethyl group which forms acetal with the adjoining oxygen atom, or $Q^1$ is a substituted alkoxymethyl group which forms acetal with the adjoining oxygen atom, when $Q^5$ is an acyl group.

Detailed explanation of the scheme F is as follows:

Mono protection of the diol [XIII] into the compound [XXXVII] can be accomplished by the same procedure as used in the synthesis of the compound [XIV] from the diol [XV] or in the synthesis of the compound [XVIII] from the compound [XVII], or by continuous extraction from the aqueous solution of the diol [XIII] containing acetic acid in the presence of an acid (e.g. sulfuric acid) with a non-polar solvent (e.g. benzene, hexane) at a temperature in the range from 0°C to 50°C.

The compound [XXXVII] can be converted into the aldehyde [XXXVIII] by the same procedure as used in the synthesis of the aldehyde [XV] from the compound [XIV].

The aldehyde [XXXVIII] can be converted into the ester [XL] by the same procedure as used in the synthesis of the compound [XVI] from the aldehyde [XV].

The ester [XL] can be converted into the alcohol [XXV] by conventional procedures.

The alcohol [XXV] can be converted into the aldehyde [XXVI] by the same procedure as used in the synthesis of the aldehyde [XV] from the alcohol [XIV].

The aldehyde [XXVI] can be converted into the carbonyl compound [IIIa] which is part of the carbonyl compound [III], by the same procedure as used in the synthesis of the carbonyl compound [IIa] from the aldehyde [IX].

And the carbonyl compound [IIIa] can be also prepared in the following way.

The aldehyde [XXXVIII] can be converted into the compound [XXVII] by the same procedure as used in the synthesis of the carbonyl compound |IIa.] from the aldehyde [IX].

The compound [XXVII] can be converted into the alcohol [XXVIII] by conventional procedures.

The alcohol [XXVIII] can be converted into the aldehyde [XXIX] by the same procedure as used in the synthesis of the aldehyde [XV] from the alcohol [XIV].

The aldehyde [XXIX] can be converted into the carbonyl compound [IIIa] by the same procedure as used in the synthesis of the ester [XVI] from the aldehyde [XV].

The carbonyl compound [IIIb], which is included within the carbonyl compound [III], can be prepared in the following way.

The reduction of the compound [XXVII] into the alcohol [XXX] can be accomplished by the same procedure as used in the synthesis of the unsaturated fatty acid [I] from the carbonyl compound [II].

The compound [XXX] can be converted into the compound [XXXI] by the same proceudre as used in the synthesis of the compound [XIV] from the diol [XIII], when $Q^1$ is an acyl group in the formula [XXX], or by the same procedure as used in the synthesis of the compound [XVIII] from the compound [XVII], when $Q^1$ is a substituted alkoxymethyl group.

The compound [XXXI] can be converted into the compound [XXXII] by conventional procedures.

The compound [XXXII] can be converted into the aldehyde [XXXIII] by the same procedure as used in the synthesis of the aldehyde [XV] from the alcohol [XIV].

The aldehyde [XXXIII] can be converted into the carbonyl compound [IIIb] by the same procedure as used in the synthesis of the ester [XVI] from the aldehyde [XV].

The aldehyde [V] used as an intermediate in the present invention can be prepared by the following scheme:

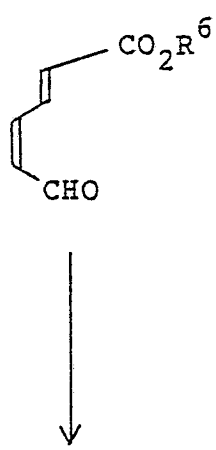

[XXXIV]

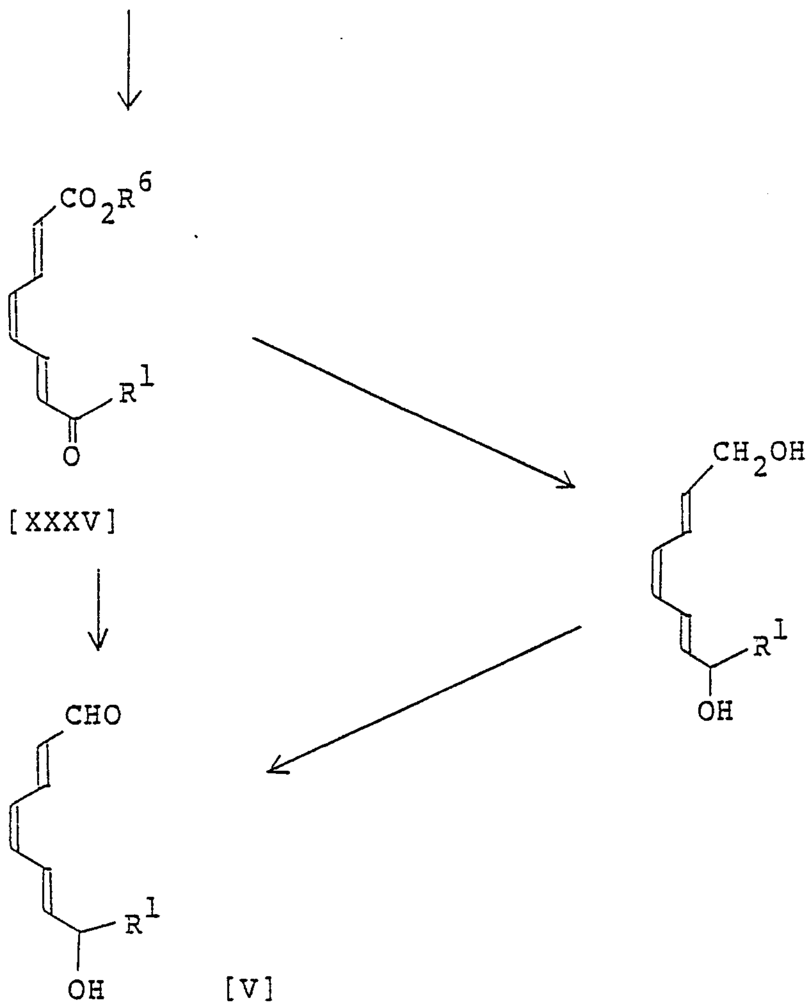

[XXXV]

[XXXVI]

[V]

Scheme G

In the formulas illustrated in the scheme G, $R^6$ is a $C_1$-$C_4$ alkyl group, $R^1$ is as defined above.

Detailed explanation of the scheme G is as follows;

Wittig reaction of the aldehyde [XXXIV] into the compound [XXXV] can be carried out by the same procedure as used in the synthesis of the carbonyl compound [IIa] from the aldehyde [IX].

Partial reduction of the ester [XXXV] into the aldehyde [V] can be accomplished by the same procedure as used in the synthesis of the aldehyde [X] from the ester [XXIII].

Reduction of the ester [XXXV] into the alcohol [XXXVI] can be carried out by the same procedure as used in the synthesis of the alcohol [XXIV] from the ester [XXIIIa].

The alcohol [XXXVI] can be converted into the aldehyde [V] by the same procedure as used in the synthesis of the aldehyde [Xa] from the alcohol [XXIV].

According to the present invention, the two stereoisomers of the formulas:

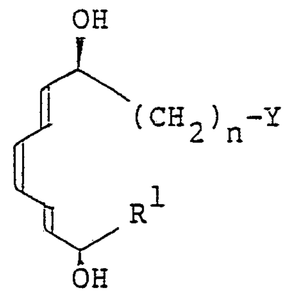

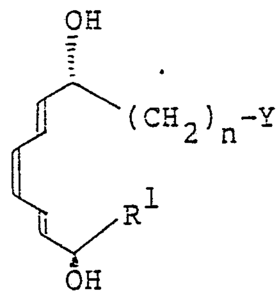

which include their corresponding enantiomers, can be prepared.

In general, the unsaturated fatty acid [I] can be obtained as a mixture of these stereoisomers, which can be easily separated by a conventional method (e.g. HPLC) with high purity.

Furthermore, unsaturated fatty acid [I] can be separated into optical isomers by a conventional method.

Among the unsaturated fatty acids [I] thus obtained, the compound [Ia] can be converted to its pharmaceutically acceptable salt form. The pharmaceutically acceptable salts of these unsaturated fatty acids are those with pharmaceutically acceptable metal cation such as sodium, potassium, magnesium and calcium, ammonium or amine cations.

The preparation of pharmaceutical compositions can be carried out by conventional methods. For example, the unsaturated fatty acids [I] may be mixed with carriers, diluents, lubricants, fillers and/or binders such as lactose, sucrose, calcium phosphate, starch, talcum, casein, magnesium stearate, methyl cellulose, polyglycols, tragacanth and the like, sometimes together with stabilizers and emulsifying agents. The resulting mixture may be processed in a usual manner to tables, capsules, pills, ampoules, ointment and the like.

In a clinical practice, the unsaturated fatty acids [I] can be administered orally, intranasally, subcutaneously, intravenously, intramuscularly, intradermally or the like.

The daily dosage may vary depending upon the administration route and the usual oral dosage of the active ingredient is between about 0.1 mg and about 100 mg daily for human beings.

Specific examples of the unsaturated fatty acid [I] are as follows:

o (6E, 8Z, 10E)-5,12-dihydroxy-nonadeca-6,8,10-trienoic acid
o (6E,8Z,10E,15Z)-methyl 5,12-dihydroxy-eicosa-6,8,10,15-tetraenoate
o (6E,8Z,10E)-N,N-dimethyl-5,12-dihydroxy-eicosa-6,8,10-triene-14-ynamide
o (6E,8Z,10E)-methyl 5,12-dihydroxy-12-cyclohexyl-dodeca-6,8,10-trienoate
o (6E,8Z,10E)-5,12-dihydroxy-12-(m-chlorophenyl)-dodeca-6,8,10-trienoic acid
o (6E,8Z,10E)-methyl 5,12-dihydroxy-12-(p-trifluoromethylphenyl)-dodeca-6,8,10-trienoate
o (6E,8Z,10E)-5,12-dihydroxy-13-cyclohexyl-trideca-6,8,10-trienoic acid
o (6F,8Z,10E)-5,12-dihydroxy-13-phenoxy-trideca-6,8,10-trienoic acid
o (7E,9Z,11E)-methyl 6,13-dihydroxy-13-cyclopentyl-trideca-7,9,11-trienoate
o (7E,9Z,11E)-methyl 6,13-dihydroxy-14-(m-chlorophenoxy)-tetradeca-7,9,11-trienoate
o (7E,9Z,11E)-N,N-diethyl-6,13-dihydroxy-15-(p-dimethylaminophenyl)-pentadeca-7,9,11-trienamide
o (5E,8Z,9E)-4,11-dihydroxy-11-cyclopentyl-undeca-5,7,9-trienamide
o (5E,7Z,9E)-N,N-dimethyl-4,11-dihydroxy-12-cyclohexyldodeca-5,7,9-trienamide
o (5E,7Z,9E)-methyl 4,11-dihydroxy-nonadeca-5,7,9-trienoate
o (5E,7Z,9E)-N-(n-butyl)-4,11-dihydroxy-12-methyl-hexadeca-5,7,9-triene-14-ynamide
o (5E,7Z,9E)-4,11-dihydroxy-13-ethoxy-trideca-5,7,9-trienoic acid
o (5E,7Z,9E)-N-benzyl-4,11-dihydroxy-12-phenyl-dodeca-5,7,9-trienamide
o (5E,7Z,9E)-methyl 4,11-dihydroxy-13-(3,4-dichlorophenyl)-trideca-5,7,9-trienoate
o (5E,7Z,9E)-4,11-dihydroxy-14-(p-methylphenyl)-tetradeca-5,7,9-trienoic acid
o (5E,7Z,9E)-N,N-tetramethylene-4,11-dihydroxy-13-(p-dimethylaminophenyl)-trideca-5,7,9-trienamide
o (5E,7Z,9E)-N-phenyl-4,11-dihydroxy-13-(p-methoxyphenyl)-trideca-5,7,9-trienamide
o (5E,7Z,9E)-4,11-dihydroxy-15-(3,4,5-trimethoxyphenyl)-pentadeca-5,7,9-trienamide
o (5E,7Z,9E)-N-ethyl-4,11-dihydroxy-15-dimethylamino-pentadeca-5,7,9-trienamide

o (5E,7Z,9E)-N,N-dimethyl-4,11-dihydroxy-13-(m-hydroxyphenyl)-trideca-5,7,9-trienamide
o (5E,7Z,9E)-ethyl 4,11-dihydroxy-11-phenyl-undeca-5,7,9-trienoate
o (5E,7Z,9E)-N,N-tetramethylene-4,11-dihydroxy-13-(p-methylthiophenyl)-trideca-5,7,9-trienamide
o (5E,7Z,9E)-N-ethyl-4,11-dihydroxy-13-(2-pyridyl)-trideca-5,7,9-trienamide
o (5E,7Z,9E)-N,N-tetramethylene-4,11-dihydroxy-13-(p-methoxyphenyl)-trideca-5,7,9-trienamide
o (5E,7Z,9E)-N,N-dimethyl-4,11-dihydroxy-13-(p-methoxyphenyl)-trideca-5,7,9-trienamide
o (5E,7Z,9E)-N,N-pentamethylene-4,11-dihydroxy-13-(p-methoxyphenyl)-trideca-5,7,9-trienamide
o (5E,7Z,9E)-N,N-dimethyl-4,11-dihydroxy-nonadeca-5,7,9-trienamide
o (6E,8Z,10E)-N,N-dimethyl-5,12-dihydroxy-eicosa-5,7,9-trienamide
o (5E,7Z,9E)-N,N-dimethyl-4,11-dihydroxy-13-(3,4-dimethoxyphenyl)-trideca-5,7,9-trienamide
o (6E,8Z,10E)-N,N-dimethyl-5,12-dihydroxy-14-(3,4-dimethoxyphenyl)-tetradeca-6,8,10-trienamide
o (6E,8Z,10E)-N,N-dimethyl-5,12-dihydroxy-14-(p-methoxyphenyl)-tetradeca-6,8,10-trienamide
o (6E,8Z,10)-N,N-tetramethylene-5,12-dihydroxy-14-(p-methoxyphenyl)-tetradeca-6,8,10-trienamide
o (5E,7Z,9E)-N,N-dimethyl-4,11-dihydroxy-henicosa-5,7,9-trienamide
o (5E,7Z,9E)-N,N-dimethyl-4,11-dihydroxy-13,17-dimethyl-octadeca-5,7,9,17-tetraenamide
o (5E,7Z,9E)-N,N-dimethyl-4,11-dihydroxy-12-methyl-trideca-5,7,9-trienamide
o (6E,8Z,10E)-N,N-dimethyl-5,12-dihydroxy-14-methyl-octadeca-6,8,10-trienamide

Practical and preferred embodiments of the present invention are illustrated in the following examples, which are not intended to limit the scope of the invention.

Referential Example 1

Methyl 5-(triphenylphosphoranylidene)levulinate (35 g) was added to a N,N-dimethylformamide solution (100 ml) of (2Z)-4-tetrahydropyranoxy-2-butenal (13 g), and the mixture was stirred at 50°C for 3 hrs. The reaction mixture was poured into water and extracted with ethyl acetate. The organic layer was dried, concentrated, and chromatographed on silica gel to give (5E,7Z)-methyl-9-tetrahydropyranoxy-4-oxo-nona-5,7-dienoate (8 g). NMR ($CDCl_3$) δ ppm

1.6 (6H, br), 2.67 (2H, t, J=6Hz),
2.90 (2H, t, J=6Hz), 3.71 (3H, s),
4.4 (2H, m), 4.70 (1H, br. s), 5.9-7.7 (4H, m)

Referential Example 2

p-Toluenesulfonic acid monohydrate (200 mg) was added to a methanol solution (150 ml) of (5E,7Z)-methyl 9-tetrahydropyranoxy-4-oxo-nona-5,7-dienoate (9 g), and the mixture was stirred at 50°C for 1 hr. Methanol was evaporated, and to the mixture was added an aqueous solution of sodium bicarbonate. Then the mixture was extracted with ethyl acetate. The extract was dried and concentrated to give al oil. Then, to a chloroform solution (100 ml) of the above oil, was added active manganese dioxide (20 g). The reaction mixture was stirred at room temperature for 10 hrs, and filtrated on celite, concentrated and chromatographed on silica gel to give (5E,7Z)-methyl 8-formyl-4-oxo-octa-5,7-dienoate (4.0 g). NMR ($CDCl_3$) δ ppm

2.70 (2H, t, J=6Hz), 2.98 (2H, t, J=6Hz),
3.70 (3H, s), 6.4-7.5 (4H, m), 10.28
(1H, d, J=7Hz)

Referential Example 3

To a tetrahydrofuran suspension (500 ml) of sodium hydride (60% dispersion in mineral oil; 3.5 g), was added dimethyl [2-oxo-4-(p-methoxyphenyl)butyl]phosphonate (24.9 g), and then to the reaction mixture, was added a tetrahydrofuran solution (100 ml) of (5E, 7Z)-methyl-4-oxo-octa-5,7-dienoate (16.6 g) at 0°C. The reaction mixture was stirred, extracted with ethyl acetate. The extract was dried, concentrated and chromatographed on silica-gel to give (5E, 7Z, 9E)-methyl 4,11-dioxo-13-(p-methoxyphenyl)-trideca-5,7,9-trienoate (12.0 g) NMR ($CDCl_3$) δ ppm
2.63 (2H, t, J=6Hz), 2.87 (6H, m),
3.67 (3H, s), 3.76 (3H, s), 6.1-7.4 (10H, m)

Example 1

Sodium borohydride (1.0 g) was added to a methanol solution (200 ml) of (5E,7Z,9E)-methyl 4,11-dioxo-13-(p-methoxyphenyl)-trideca-5,7,9-trienoate (9.0 g) at 0°C; and the mixture was stirred at 0°C for 2 hrs. Then, to this mixture was added pyrrolidine (15 ml). The resulting mixture was stirred at room temperature for 4 hr., poured into water and extracted with ethyl acetate. The extract was dried, concentrated and chromatographed on silica gel to give (5E,7Z,9E)-N,N-tetramethylene-4,11-dihydroxy-13-(p-methoxyphenyl)-trideca-5,7,9-trienamide (7.0 g). NMR ($CDCl_3$) δ ppm
1.9 (4H, m), 2.45 (2H, t, J=7Hz),
2.66 (2H, t, J=7Hz), 3.4 (4H, m),
3.79 (3H, s), 4.3 (2H, br), 5.7-6.8 (6H, m),
6.83 (2H, d, J=8Hz), 7.11 (2H, d, J=8Hz)

Example 2

According to the same procedure as that of Example 1, there were obtained the following compounds.

o (5E,7Z,9E)-N,N-dimethyl-4,11-dihydroxy-nonadeca-5,7,9-trienamide
NMR ($CDCl_3$) δ ppm
0.88 (3H, t, J=7Hz), 2.49 (2H, t, J=6Hz),
2.97 (3H, s), 3.02 (3H, s), 4.18 (1H, q, J-7Hz),
4.31 (1H, br), 5.73 (2H, m), 5.98 (2H, m),
6.73 (2H, m)

o (6E,8Z,10E)-N,N-dimethyl-5,12-dihydroxy-eicosa-6,8,10-trienamide
NMR ($CDCl_3$)
0.88 (3H, t, J=7Hz), 2.36 (2H, br)
2.96 (3H, s), 3.01 (3H, s), 4.10 (2H,m),
5.75 (2H, m), 5.97 (2H, d, J=10Hz),
6.69 (2H, m)

o (5E,7Z,9E)-N,N-dimethyl-4,11-dihydroxy-henicosa-5,7,9-trienamide
$^1$H NMR ($CDCl_3$) δ ppm
0.88 (3H, t, J=7Hz), 1.3 (18H, m), 2.98 (3H, s),
3.03 (3H, s), 4.2 (2H, br), 5.8-6.7 (6H, m)

o (5E,7Z,9E)-N,N-dimethyl-4,11-dihydroxy-13,17-dimethyl-octadeca-5,7,9,17-tetraenamide
$^1$H NMR ($CDCl_3$) δ ppm
0.92 (2H, d, J=7Hz), 1.60 (3H, s), 1.68 (3H, s),
2.79 (3H, s), 3.01 (3H, s), 4.2 (2H, br),
5.8-6.8 (6H, m)

o (5E,7Z,9E)-N,N-dimethyl-4,11-dihydroxy-12-methyl-trideca-5,7,9-trienamide
$^1$H NMR ($CDCl_3$) δ ppm
1.13 (6H, d, J-7Hz), 2.97 (3H, s), 3.02 (3H, s),
4.2 (2H, m), 5.8-6.9 (6H, m)

o (6E,8Z,10E)-N,N-dimethyl-5,12-dihydroxy-14-methyl-octadeca-6,8,10-trienamide
$^1$H NMR ($CDCl_3$) δ ppm
0.91 (3H, t, J=7Hz), 0.91 (3H, t, J=6Hz),
2.98 (3H, s), 3.03 (3H, s), 4.2 (2H, br),
5.7-6.8 (6H, m)

## Claims

1. A compound of the formula:

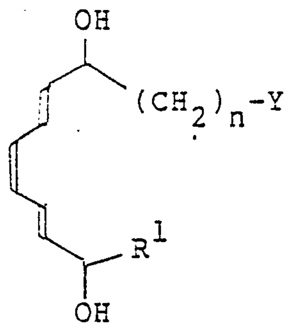

[I]

wherein Y is a free or esterified carboxyl group, or a group of the formula:

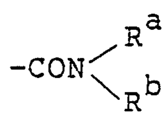

(wherein $R^a$ and $R^b$ are each independently a hydrogen atom, a $C_1$-$C_4$ alkyl group, a $C_3$-$C_7$ cycloalkyl group, a benzyl group, a phenyl group, a phenyl group substituted with a halogen atom or a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, a $C_4$-$C_{10}$ cycloalkenyl group, a hydroxy $C_1$-$C_{12}$ alkyl group, a $C_1$-$C_{12}$ alkyl group substituted with a group of the formula:

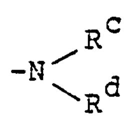

(wherein $R^c$ and $R^d$ are each independently a hydrogen atom, or a $C_1$-$C_4$ alkyl group), a $C_3$-$C_{10}$ heterocyclic group, a phenyl group optionally substituted with one to three substituents selected from the group consisting of halogen atom, hydroxyl group, $C_1$-$C_4$ alkyl group, trifluoromethyl group, $C_1$-$C_4$ alkoxy group, and group of the formula:

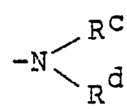

wherein $R^c$ and $R^d$ are as defined above) or a group of the formula: A-B [wherein A is a $C_1$-$C_7$ alkylene chain and B is a $C_3$-$C_{10}$ cycloalkyl group, a $C_4$-$C_{10}$ cycloalkenyl group, a $C_1$-$C_{12}$ alkoxy group, a $C_1$-$C_{12}$ alkylthio group, a $C_3$-$C_{10}$ cycloalkoxy group, a $C_4$-$C_{10}$ cycloalkenyloxy group, a $C_3$-$C_{10}$ heterocyclic group, or a phenyl or phenoxy group optionally substituted with one to three substituents selected from the group consisting of halogen atom, hydroxy group, $C_1$-$C_4$ alkyl group, group of the formula:

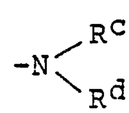

(wherein $R^c$ and $R^d$ are as defined above), trifluoromethyl group, $C_1$-$C_4$ alkylthio group and $C_1$-$C_4$ alkoxy group]; n is 2, 3 or 4, or a pharmaceutically acceptable salt thereof.

2. The compound according to Claim 1, wherein Y is a group of the formula:

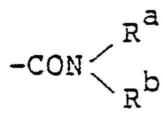

(wherein $R^a$ and $R^b$ are each independently a hydrogen atom, a $C_1$-$C_4$ alkyl group, or, when taken together with the adjacent nitrogen atom, they represent a 5 to 7 membered saturated heterocyclic group); $R^1$ is a $C_1$-$C_{12}$ alkyl group, a $C_2$-$C_{12}$ alkenyl group, a $C_2$-$C_{12}$ alkynyl group, a $C_3$-$C_{10}$ cycloalkyl group, or a group of the formula: A-B [A is a $C_1$-$C_7$ alkylene chain and B is a $C_3$-$C_{10}$ cycloalkyl group, a $C_1$-$C_{12}$ alkoxy group, or a phenyl or phenyl group optionally substituted with one to three substituents selected from the group consisting of halogen atom, $C_1$-$C_4$ alkyl group, group of the formula :

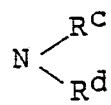

(wherein $R^c$ and $R^d$ are each independently a $C_1$-$C_4$ alkyl group), trifluoromethyl group, and $C_1$-$C_4$ alkoxy group].

3. The compound according to Claim 1 wherein $R^1$ is a group of the formula: A-B [A is a $C_1$-$C_7$ alkylene chain and B is a phenyl group optionally substituted with one to three substituents selected from the group consisting of halogen atom, $C_1$-$C_4$ alkyl group, group of the formula:

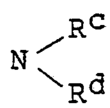

(wherein $R^c$ and $R^d$ are each independently a $C_1$-$C_4$ alkyl group), trifloromethyl group, and $C_1$-$C_4$ alkoxy group], or a pharmaceutically acceptable salt thereof.

4. The compound according to Claim 2 wherein $R^1$ is a group of the formula: A-B [A is a $C_1$-$C_7$ alkylene chain and B is a phenyl group optionally substituted with one to three substituents selected from the group consisting of halogen atom, $C_1$-$C_4$ alkyl group, group of the formula:

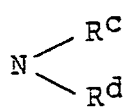

(wherein $R^c$ and $R^d$ are each independently a $C_1$-$C_4$ alkyl group), trifluoromethyl group, and $C_1$-$C_4$ alkoxy group].

5. The compound according to Claim 1, wherein Y is a group of the formula:

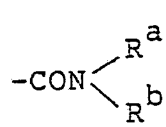

wherein $R^a$ and $R^b$ are each independently a $C_1$-$C_4$ alkyl group or, when taken together with the adjacent nitrogen atom, they represent a 5 to 7 membered saturated heterocyclic group; $R^1$ is a $C_1$-$C_{12}$ alkyl group, a $C_2$-$C_{12}$ alkenyl group or a group of the formula:

A-B

wherein A is a $C_1$-$C_7$ alkylene chain and B is a phenyl group optionally substituted with a $C_1$-$C_4$ alkoxy group; and n is 2 or 3.

6. The compound according to Claim 5, wherein Y is a group of the formula:

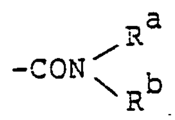

wherein $R^a$ and $R^b$ are each independently a $C_1$-$C_4$ alkyl group.

7. The compound according to Claim 5, wherein Y is a group of the formula:

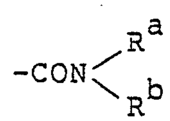

wherein $R^a$ and $R^b$ are taken together with the adjacent nitrogen atom to represent a 5 to 7 membered saturated heterocyclic group selected from a member consisting of a pyrrolidinyl group, a piperidinyl group and a homopiperidinyl group.

8. The compound according to Claim 5, wherein $R^1$ is a $C_1$-$C_{12}$ alkyl group selected from a member consisting of a isopropyl group, a n-octyl group, a n-decyl group and a 2-methylhexyl group.

9. The compound according to Claim 5, wherein $R^1$ is a $C_2$-$C_{12}$ alkenyl group selected from a member consisting of a 5-heptenyl group, a 6-methyl-5-heptenyl group and a 2,6-dimethyl-5-heptenyl group.

10. The compound according to Claim 5, wherein $R^1$ is a phenethyl group optionally substituted with a $C_1$-$C_4$ alkoxy group.

11. (5E,7Z,9E)-N,N-tetramethylene-4,11-dihydroxy-13-(p-methoxyphenyl)-trideca-5,7,9-trienamide.

12. (5E,7Z,9E)-N,N-dimethyl-4,11-dihydroxy-nonadeca-5,7,9-trienamide.

13. (6E,8Z,10E)-N,N-dimethyl-5,12-dihydroxy-eicosa-6,8,10-trienamide.

14. (5E,7Z,9E)-N,N-dimethyl-4,11-dihydroxy-henicosa-5,7,9-trienamide.

15. (5E,7Z,9E)-N,N-dimethyl-4,11-dihydroxy-13,17-dimethyl-octadeca-5,7,9,17-tetraenamide.

16. (5E,7Z,9E)-N,N-dimethyl-4,11-dihydroxy-12-methyl-trideca-5,7,9-trienamide.

17. (6E,8Z,10E)-N,N-dimethyl-5,12-dihydroxy-14-methyl-octadeca-6,8,10-trienamide.

18. A process for producing a compound as claimed in Claim 1 or a pharmaceutically acceptable salt thereof, which comprises reacting a compound of the formula:

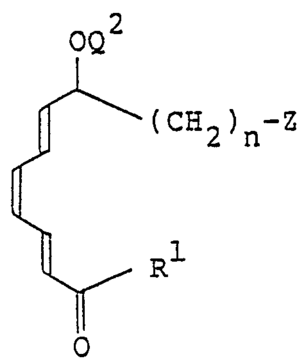

wherein $R^1$ and n are as defined in Claim 1; $Q^2$ is a hydrogen atom or an acyl group; and Z is an esterified carboxyl group or a group of the formula:

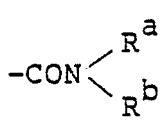

(wherein $R^a$ and $R^b$ are as defined in Claim 1), with a reducing agent optionally followed by deprotection of a protected hydroxyl group, hydrolysis of an ester group, esterification of a carboxyl group, amidation of a free or esterified carboxyl group, and/or transesterification.

19. A process for producing a compound as claimed in Claim 1 or a pharmaceutically acceptable salt thereof, which comprises reacting a compound of the formula:

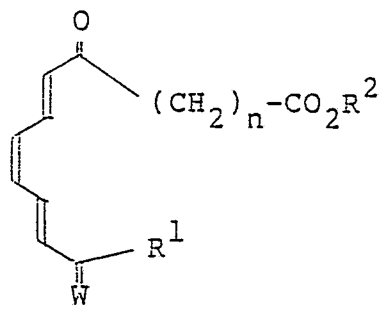

wherein $R^1$ and n are each as defined in Claim 1, and $R^2$ is a $C_1$-$C_4$ alkyl group, W is an oxygen atom, or a group of the formula:

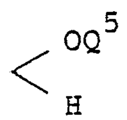

($Q^5$ is an acyl group or a substituted alkoxymethyl group which forms acetal with the adjoining oxygen atom), with a reducing agent optionally followed by deprotection of a protected hydroxyl group, hydrolysis of an ester group, esterification of a carboxyl group, amidation of a free or esterified carboxyl group, and/or transesterification.

20 A compound of the formula:

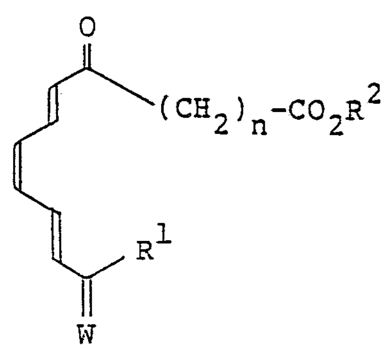

wherein $R^1$, $R^2$, W and n are each as defined in Claim 19.

21. A pharmaceutical composition useful as an anti-leucotriene $B_4$ drug, which comprises an effective amount of a compound as claimed in any one of Claims 1 to 17 or a pharmaceutically acceptable salt thereof as an active ingredient and a pharmaceutically acceptable carrier or diluent.

22. A compound of Claim 1 or a pharmaceutically acceptable salt thereof for use as an active therapeutic substance.

23. A compound of any one of Claims 1 to 17 or a pharmaceutically acceptable salt thereof for use in a method of treating inflammatory or allergic states.